Europäisches Patentamt

(19) European Patent Office

Office européen des brevets

(11) Publication number: **0 302 420 B1**

## (12) EUROPEAN PATENT SPECIFICATION

(45) Date of publication of patent specification: **17.06.92**    (51) Int. Cl.5: **A61L  2/20**

(21) Application number: **88112384.8**

(22) Date of filing: **29.07.88**

(54) **Low pressure hydrogen peroxide vapor sterilization system.**

(30) Priority: **30.07.87 US 79558**

(43) Date of publication of application:
**08.02.89 Bulletin  89/06**

(45) Publication of the grant of the patent:
**17.06.92 Bulletin  92/25**

(84) Designated Contracting States:
**AT BE CH DE ES FR GB IT LI LU NL SE**

(56) References cited:
**WO-A-88/04939**
**GB-A- 2 105 591**
**US-A- 4 169 124**
**US-A- 4 230 663**
**US-A- 4 437 567**

(73) Proprietor: **JOHNSON & JOHNSON MEDICAL, INC. (a New Jersey corp.)**
**One Johnson & Johnson Plaza**
**New Brunswick, New Jersey 08933(US)**

(72) Inventor: **Jacobs, Paul T.**
**2815 Oak Trail Court**
**Arlington Texas 76016(US)**
Inventor: **Lin, Szu-Min**
**405 Betsy Ross Drive**
**Arlington Texas 76018(US)**
Inventor: **Addy, Tralance O.**
**1904 Rockcliff Court**
**Arlington Texas 76012(US)**

(74) Representative: **Strehl, Schübel-Hopf, Groening**
**Maximilianstrasse 54 Postfach 22 14 55**
**W-8000 München 22(DE)**

**Description**

BACKGROUND OF THE INVENTION

Field of the Invention

The present invention relates to the vapor sterilization of articles and, more particularly to the use of hydrogen peroxide vapor at very low pressures to kill micro-organisms on objects such as medical instruments.

Description of the Prior Art

Various methods of sterilization have been used in the past for the sterilization of articles such as disposable and reusable medical supplies and equipment, foods and food containers. Sterilization by steam or by dry heat has been extensively used in the past but is not useful to sterilize materials that are adversely affected by high temperatures and/or moisture. Ethylene oxide gas has also been used but suffers from the drawback that it may leave toxic residues on the sterile articles. Extended aeration cycles may be required to reduce residual ethylene oxide from some articles making the ethylene oxide sterilization process excessively long.

Hydrogen peroxide is known to have bactericidal properties and has been used in solution to kill bacteria on various surfaces. U.S. Patent No. 4,437,567 discloses the use of aqueous hydrogen peroxide solutions at low concentrations, i.e., 0.01% to 0.10% by weight, to sterilize packaged products for medical or surgical use. At room temperature, such sterilization requires at least 15 days to be effective. At higher temperatures, sterilization can be accomplished in approximately one day.

U.S. Patent Nos. 4,169,123; 4,169,124 and 4,230,663 disclose the use of hydrogen peroxide vapor at temperatures below 80°C and concentrations of 0.10 to 75 mg $H_2O_2$ vapor/liter for sterilization and disinfection. These patents also disclose the use of reduced pressure such as negative 15 or 25 inches of mercury (381 or 635 torr/506.73 or 844.5 mbar) in sterilization processes utilizing hydrogen peroxide vapor. Depending upon concentration and temperature, sterilization times are reported to vary from 30 minutes to four hours.

U.S. Patent No. 4,643,876 discloses a plasma sterilization process which employs hydrogen peroxide vapor as the precursor for the active species generated during the plasma generation cycle and employs a pre-treatment cycle prior to the plasma generation cycle.

U. S. Patent No. 4,512,951 discloses a hydrogen peroxide liquid film sterilization method. In the disclosed method the sterilization chamber is evacuated to an absolute pressure of between 2 and 4 inches of mercury (50.8 and 101 torr/67.56 and 134.33 mbar) before the hydrogen peroxide is vaporized into the chamber. The hydrogen peroxide is then introduced into the chamber and condensed on the surface of the items to be sterilized by maintaining the temperature of the items below the dew point of the vapor mixture.

While some of the prior art vapor sterilization methods have commercial promise, they are often associated with significant problems related to one or more of the factors involving efficacy, convenience, safety, and technical or economic practicality. For instance, a well known and obvious method of increasing the sporicidal activity of a sterilant is to increase the concentration of the sterilant in the sterilization chamber. Although such an approach may shorten the time for sterilization, the higher concentration of sterilant may have deleterious effects on some materials, and may also necessitate vigorous and cumbersome methods to remove residuals after the sterilization process is complete. For example, hydrogen peroxide is an oxidizing agent, and at high concentrations, degradation or bleaching of certain materials may occur. In some of the prior art processes as described in the aforementioned patents, sterilization is carried out at reduced pressures (between approximately 50 and 100 torr/66.5 and 133 mbar) to improve vapor diffusion and thereby reduce the concentration of sterilant and/ or time necessary for sterilization. Even under these conditions, a high concentration of sterilant or elevated temperatures may still be necessary to enhance the process.

In U.S. Patent No. 4,512,951, an attempt is made to improve the sporicidal activity of hydrogen peroxide vapor by condensing heated vapor as a liquid film on the surfaces of the items to be sterilized and maintaining the liquid film until sterilization is complete. The patent also discloses that "temperatures generally within the range of 15°C to 55°C effect sterilization of most articles within hours." While the increased concentration of hydrogen peroxide in the condensate may somewhat enhance sterilization, it is clear that at least some surfaces would tend to absorb high levels of hydrogen peroxide and may require additional means for removal, such as aeration, thereby extending the interval between successive uses of the items to be sterilized.

It is accordingly an object of the present invention to overcome the limitations of the prior art vapor and vapor condensate sterilization processes by providing a method where sterilization is carried out in the vapor phase at ambient or relatively low temperatures, and at low concentrations of a sterilant such as hydrogen peroxide, is accomplished within a short period of time, and results in a minimum of sterilant residue on the sterilized articles.

Summary of the Invention

The present invention involves the use of hydrogen peroxide in a very low pressure vapor sterilization system. The process is carried out by placing the article to be sterilized in a chamber, evacuating the chamber to a pressure below about 1.33 mbar (1.0 torr) and preferably of from about 66.5 $\mu$bar to 0.13 mbar (0.05 to 0.1 torr) introducing aqueous hydrogen peroxide and allowing at least a portion of it to vaporize to create a hydrogen peroxide atmosphere in said chamber and maintaining the pressure in the chamber below the vapor pressure of the hydrogen peroxide for a period of time sufficient to achieve the desired sterilization. Applicants have discovered that at the very low pressures of the present invention, the sporicidal activity of hydrogen peroxide is unexpectedly and dramatically enhanced so that even at room temperature and with low concentrations of hydrogen peroxide, sterilization is achieved within minutes rather than hours. It has been found that the most dramatic improvement in sterilization rate is obtained generally when the pressure in the sterilization chamber, after injection of hydrogen peroxide, is maintained below the vapor pressure of the hydrogen peroxide solution.

The present invention avoids the limitations of the prior art vapor processes by providing a sterilization method in which sterilization can be rapidly achieved at low concentration of hydrogen peroxide and without the application or generation of heat. A relatively simple apparatus can thus be employed for sterilization, and a wide range of materials can be sterilized safely by the method.

Brief Description of the Drawings

Figure 1 shows a schematic drawing of the sterilization apparatus used in the present invention.

Figure 2 is a plot of experimental data showing the effect of initial sterilizer pressure on the sporicidal activity of hydrogen peroxide.

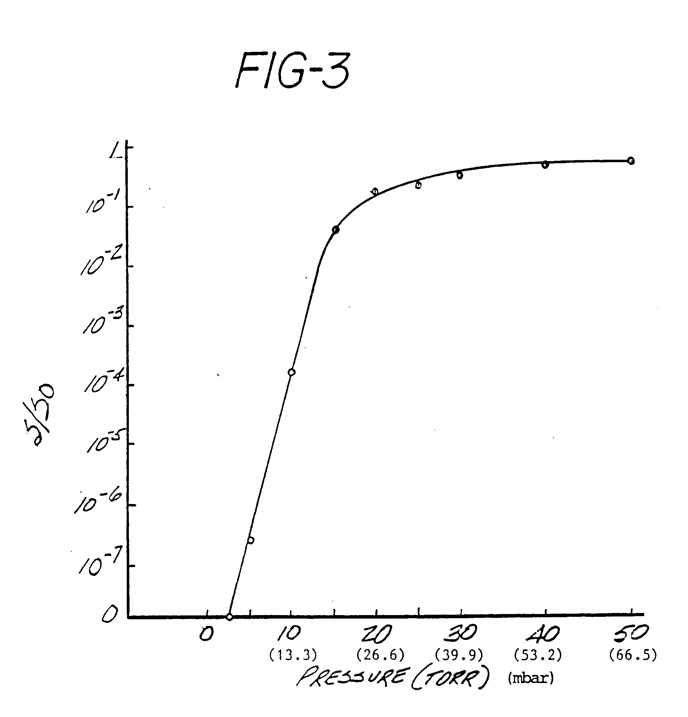

Figure 3 is a plot of experimental data showing the effect of actual sterilization pressure on the sporicidal activity of hydrogen peroxide.

Detailed Description of the Invention

The process of the present invention differs from prior art hydrogen peroxide sterilization processes in several important respects. First, the sterilization chamber is initially evacuated to a very low pressure which is significantly lower than the pressure generally used in prior art vapor sterilization processes. As will be described later, this very low initial pressure results in a significant enhancement of sporicidal activity. Secondly, in contrast to prior art processes which rely on an increase in temperature or on the concentration of the hydrogen peroxide sterilant in a liquid film to accelerate the sterilization process, the process of the present invention is conducted at room temperature and entirely in the vapor phase. An added advantage of the present process is that, with the low concentration of hydrogen peroxide involved and the maintenance of the sterilant in a vapor phase, residuals of hydrogen peroxide on the items to be sterilized are minimized. Furthermore, the process of the present invention can achieve sterilization in minutes rather than hours, without the application of heat anywhere in the process. It is, of course, recognized that sterilization may nevertheless be accelerated through the application of heat, although a major advantage of the process is that it may be carried out at relatively low temperatures, normally below about 40°C.

In accordance with the process of the present invention, the article to be sterilized is placed in a chamber, the chamber is closed and a vacuum is drawn to reduce the pressure within the chamber to less than about 1.33 mbar (1.0 torr) and most preferably to a pressure of from about 66.5 $\mu$bar to 0.13 mbar (0.05 to 0.1 torr). An aqueous solution of hydrogen peroxide is injected into the evacuated chamber and allowed to vaporize, whereby the article to be sterilized is contacted with hydrogen peroxide vapor while the pressure of the chamber is maintained below about 39.9 mbar (30 torr) and most preferably below 19.95 mbar (15 torr). The hydrogen peroxide vapor is maintained in contact with the article to be sterilized for a period of time sufficient to allow complete sterilization.

The articles to be sterilized by the present process may be packaged in various materials commonly employed to package products which are to be sterilized by a gaseous medium as for example, by ethylene oxide. The preferred materials are gas permeable spunbonded polyethylene commonly available under the trademark "TYVEK"[R] or composites of "TYVEK"[R] with a polyethylene terephthalate film commonly available under the trademark "MYLAR"[R]. Other similar packaging materials may also be employed.

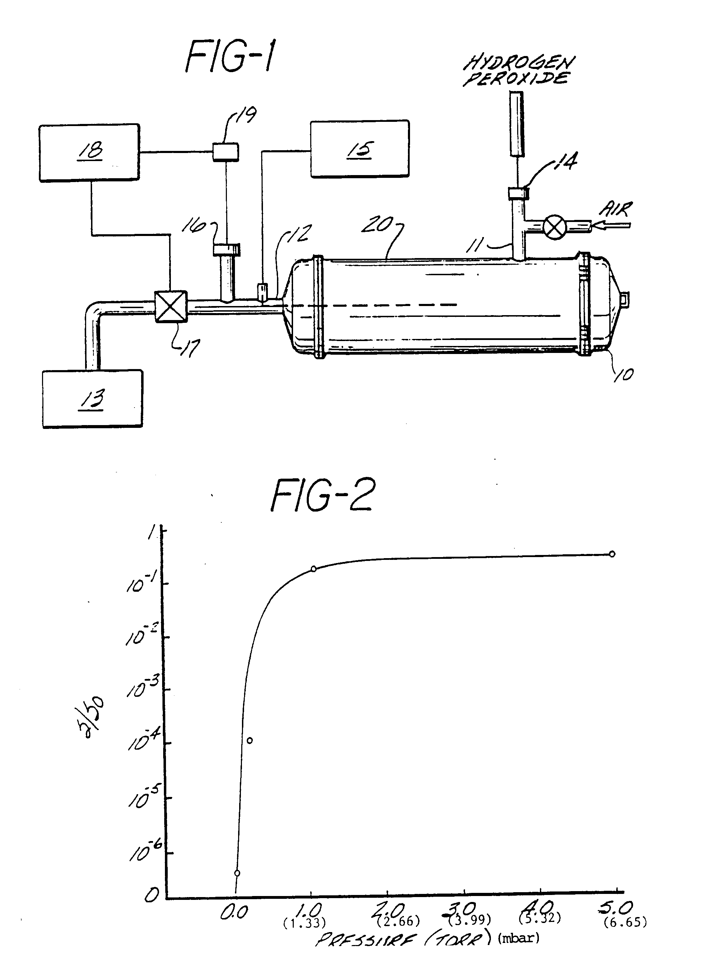

Turning now to Figure 1 there is illustrated a schematic drawing of a sterilization apparatus suitable for use in the practice of the present invention. The apparatus comprises sterilization chamber 20 which includes a hinged door 10 through which articles to be sterilized can be introduced. The chamber also includes gas inlet 11 connected to a source of hydrogen peroxide and sterile air, and outlet 12 connected to a vacuum pump 13 to enable the chamber to be evacuated. A suitable vacuum pump for use in the present process is a Leybold-Heraeus Model D8A rotary vane pump. Port 14 in gas inlet line 11 permits the introduction of an aqueous solution of hydrogen peroxide into chamber 20. The system also includes a thermo-sensor/recorder 15 to enable continuous monitoring of the temperature inside chamber. Any suitable device for sensing temperature such as a thermocouple may be used. A pressure control system consisting of a dual element transducer 16, valve 17, valve controller 18 and power supply readout 19 is disposed in line 12. The transducer monitors the pressure inside the chamber from 13.3 to 1330 mbar (10 torr to 1000 torr) with one element and from 1.33 $\mu$bar to 13.3 mbar ($10^{-3}$ torr to 10 torr) with the other element. Suitable components for the pressure control system are MKS model 222CA-01000AB and Model 222CA-00010AB transducers, a MKS Model 253A-1-40-1-SP throttle valve, a MKS Model 252A-MSO-4 valve controller and a MKS PDR-C-2C power supply readout.

As previously indicated, in the present process the solution of hydrogen peroxide is injected into a highly evacuated chamber to develop a vapor for sterilization. The hydrogen peroxide is preferably in the form of an aqueous solution containing from about 10 to 70 percent by weight hydrogen peroxide, with the most preferred concentration being from about 30% to 50%. While the results of the process improve as the concentration of hydrogen peroxide in the solution is increased, there can be significant handling problems with solutions at the higher concentrations. Accordingly, a 30% to 50% solution is effective and most preferred for use in this process. The concentration of hydrogen peroxide vapor in the chamber will normally range from 0.1 to 10 milligrams of hydrogen peroxide per liter of chamber volume. Higher concentrations of hydrogen peroxide generally result in shorter sterilization times.

The general operation of the present process is as follows:

(1) The object or article to be sterilized is placed in a vacuum chamber.

(2) The chamber is evacuated to a pressure of below about 1.33 mbar (1.0 torr), the most preferred pressure being from 66.5 $\mu$bar to 0.13 mbar (0.05 to 0.1 torr. For practical reasons associated with commercially available evacuation equipment, the latter range is a reasonable lower limit of pressure.

(3) An aqueous solution of hydrogen peroxide is injected into the chamber and allowed to vaporize and create a hydrogen peroxide atmosphere in the chamber while maintaining the chamber pressure below about 39.9 mbar (30 torr), and preferable below 26.6 mbar (20 torr). The amount of the hydrogen peroxide injected into the chamber should be sufficient to provide from about 0.1 to 10 mg of hydrogen peroxide per liter of chamber volume. The preferred concentration is from about 1 to about 5 mg $H_2O_2$/liter.

(4) The object to be sterilized is maintained in contact with the hydrogen peroxide vapor for a period of time sufficient to effect complete sterilization. In many cases, the sterilization is complete in 20 minutes or less.

The entire process is normally carried out at temperatures of less than 40°C, and in most cases the process may be carried out at room temperature. As explained above, higher temperatures will shorten the time for achieving sterilization, but the present process allows effective sterilization to be obtained at low temperatures and with short cycle times compared to prior art processes.

Upon completion of the sterilization cycle, sterile gas such as filtered, bacteria free air can be fed into the chamber through inlet 11 to raise the pressure to atmospheric levels and permit the sterilized articles to be removed. Since the sterilization process is conducted at a temperature and pressure whereby the hydrogen peroxide in contact with the article being sterilized is maintained in the vapor phase, no lengthy and complicated steps are required at the completion of the sterilization cycle to remove residual hydrogen peroxide from the sterilized article or its packaging.

In the following examples, the effectiveness of the sterilization cycle is expressed as the ratio of the number of organisms surviving the test (S) to the initial number of organisms (So) which were placed on the test specimen. All tests were conducted on Tyvek* packaged Bacillus subtilis (var. globigii) spores on paper discs. The test specimens were placed in a 12 liter chamber, the chamber was evacuated to the desired initial pressure, and an aqueous solution of hydrogen peroxide was then injected into the chamber and allowed to vaporize. After maintaining contact between the vapor and test samples for a predetermined length of time, and at a specified temperature and pressure, the vacuum in the chamber was released by admitting filtered, sterile air, and the test samples were removed and evaluated to determine the number of surviving organisms.

## EXAMPLE 1

A series of tests were conducted to determine the effect of various initial pressures on sporicidal activity in the present hydrogen peroxide sterilization system. All tests were run under the same reaction conditions except for the initial pressure which was varied as indicated in Table I.

Specifically, the effect of the initial pressure on sporicidal activity was determined by injecting a 30% aqueous solution of hydrogen peroxide into the sterilization chamber that had previously been evacuated to an initial pressure of 33.25 $\mu$bar, 0.13 mbar, 1.33 mbar and 6.65 mbar (0.025, 0.1, 1.0 and 5.0 torr). Sufficient hydrogen peroxide was injected to provide a concentration of 1.0 mg $H_2O_2$/liter in the chamber assuming total vaporization. As the hydrogen peroxide vaporized, the pressure in the system increased slightly, and an additional amount of filtered air was admitted as required to establish the sterilization pressure at 13.3 mbar (10 torr). The total sterilization time was 20 minutes and all experiments were conducted at room temperature. The results of these experiments as presented in Table 1 demonstrate that the sporicidal activity of the hydrogen peroxide vapor increased as the initial pressure in the system decreased, with the most dramatic improvement occuring as pressure was reduced below 1.33 mbar (1.0 torr).

TABLE I

| Effect of initial pressure on sporicidal activity of hydrogen peroxide vapor with Bacillus subtilis (var. globigii) spores | | | | |
|---|---|---|---|---|
| Initial Pressure | | Final Pressure | | Sporicidal Activity |
| (Torr) | (mbar) | (Torr) | (mbar) | (S/So) |
| 0.025 | (0.033) | 10 | (13.3) | 0.0 |
| 0.1 | (0.13 ) | 10 | (13.3) | $1.1 \times 10^{-4}$ |
| 1.0 | (1.33 ) | 10 | (13.3) | $1.8 \times 10^{-1}$ |
| 5.0 | (6.65 ) | 10 | (13.3) | $2.8 \times 10^{-1}$ |
| The data in Table I are depicted graphically in Figure 2. | | | | |

## EXAMPLE II

A series of experiments was carried out to demonstrate the effect of sterilization pressure on sporicidal activity with the initial pressure constant at 0.13 mbar (0.1 torr). In each case, sufficient 30% hydrogen peroxide solution was introduced to produce a vapor concentration of 1.0 mg $H_2O_2$/liter in the chamber. The $H_2O_2$ solution was allowed to vaporize and diffuse for 2 minutes, after which the pressure in the system was increased to the level desired for sterilization by admitting filtered, sterile air. In all tests, the total exposure time of the test specimens to the hydrogen peroxide vapor was 20 minutes including the two minute vaporization-diffusion time, and the experiments were conducted at room temperature. The results of these tests are shown in Table II. As illustrated by those data, significantly improved sporicidal activity was obtained at sterilization pressures below 26,6 mbar (20 torr). These pressures are less than the vapor pressure of hydrogen peroxide at room temperature, which is approximately 23.94 mbar (18 torr), and the increased sporicidal activity at these pressures may be due at least in part to more complete vaporization of hydrogen peroxide.

TABLE II

| Effect of final pressure on sporicidal activity of hydrogen peroxide vapor with Bacillus subtilis (var. globigii) spores | | | | |
|---|---|---|---|---|
| Initial Pressure | | Final Pressure | | Sporicidal Activity |
| (Torr) | (mbar) | (Torr) | (mbar) | (S/So) |
| 0.1 | (0.13) | 50 | (66.5) | $7.3 \times 10^{-1}$ |
| 0.1 | (0.13) | 40 | (53.2) | $6.9 \times 10^{-1}$ |
| 0.1 | (0.13) | 30 | (39.9) | $5.4 \times 10^{-1}$ |
| 0.1 | (0.13) | 25 | (33.25) | $3.5 \times 10^{-1}$ |
| 0.1 | (0.13) | 20 | (26.6) | $2.8 \times 10^{-1}$ |
| 0.1 | (0.13) | 15 | (19.95) | $6.5 \times 10^{-2}$ |
| 0.1 | (0.13) | 10 | (13.3) | $1.1 \times 10^{-4}$ |
| 0.1 | (0.13) | 5 | ( 6.65) | $4.2 \times 10^{-7}$ |
| 0.1 | (0.13) | 2.5 | ( 3.33) | 0.0 |
| The data in Table II are depicted graphically in Figure 3. | | | | |

EXAMPLE III

The procedure of Example II was repeated except that the sterilization temperature was raised to 40°C. In all experiments at the 40°C temperature, the observed sporicidal activity (S/So) was zero (total kill) demonstrating the positive effect of a relatively small increase in temperature on sporicidal activity under the low pressure sterilization conditions of the present invention.

EXAMPLE IV

A series of experiments was carried out to demonstrate the effect of increasing the concentration of hydrogen peroxide vapor in the sterilization process. The procedure of Example II was followed with only the amount of 30% hydrogen peroxide solution being varied at each of four sterilization pressures. Table IV presents the observed results when the concentration of hydrogen peroxide vapor was 1.0 and 2.5 mg/liter of chamber volume.

TABLE IV

| Effect of hydrogen peroxide vapor concentration on sporicidal activity with Bacillus subtilis (var. globigii) spores. | | | |
|---|---|---|---|
| Final Pressure | | 1.0 mg/l | 2.5 mg/l |
| (Torr) | (mbar) | | |
| 50 | (66.5) | $7.3 \times 10^{-1}$ | $2.2 \times 10^{-1}$ |
| 40 | (53.2) | $6.9 \times 10^{-1}$ | $1.9 \times 10^{-3}$ |
| 30 | (39.9) | $5.4 \times 10^{-1}$ | 0.0 |
| 20 | (26.6) | $2.8 \times 10^{-1}$ | 0.0 |
| These data indicate that, as expected, sporicidal activity increases as the concentration of hydrogen peroxide in the vapor is increasesd. | | | |

EXAMPLE V

A series of experiments was carried out to demonstrate the effect of exposure time or contact time in the sterilization process. In this series, the procedure of Example II was followed with only the sterilization times being varied at each of three final sterilization pressures. Table V shows the observed results when the sterilization contact times were 20 minutes and 60 minutes:

TABLE V

| Effect of sterilization time on sporicidal activity with Bacillus subtilis (var. globigii) spores. | | | |
|---|---|---|---|
| Final Pressure | | 20 Minutes | 60 Minutes |
| (Torr) | (mbar) | | |
| 50 | (66.5) | $7.3 \times 10^{-1}$ | $3.7 \times 10^{-4}$ |
| 40 | (53.2) | $6.9 \times 10^{-1}$ | 0.0 |
| 30 | (39.9) | $5.4 \times 10^{-1}$ | 0.0 |
| These data show that, as expected, the sporicidal effect increases as contact time or exposure time is increased. | | | |

The reason for the dramatic and unexpected enhancement of sporicidal activity at the very low pressure conditions of the present invention are not fully understood, but it appears that the very low initial evacuation pressure and the low pressure maintained during sterilization cause most if not all of the hydrogen peroxide sterilant solution to be vaporized. In addition, sorption and desorption phenomena on the surfaces of the spores or other microorganisms may be more important factors at low pressures, and the process may allow more sites on the spores to be available to the hydrogen peroxide vapor.

While the present invention has been described in detail and with reference to specific examples thereof, it will be apparent to one skilled in the art that various changes and modifications can be made therein without departing from the spirit and scope thereof.

**Claims**

1. A process of vapor sterilization of articles comprising the steps of:
   a) placing an article to be sterilized into a sterilization chamber,
   b) evacuating said chamber
   c) creating a hydrogen peroxide atmosphere in the chamber and allowing the hydrogen peroxide vapor to contact the article,
   d) maintaining the chamber at a low pressure for a period of time sufficient to achieve sterilization, characterized in that
   - the chamber is evacuated to a pressure below 1.33 mbar (1.0 torr),
   - the hydrogen peroxide is introduced into the chamber in the form of an aqueous solution,
   - the concentration of hydrogen peroxide vapor contacting the article is in the range of from about 0.1 to about 10 mg/l, and
   - the chamber is maintained at a temperature of less than about 40°C and a pressure below the vapor pressure of hydrogen peroxide during the sterilization process.

2. Process according to claim 1, characterized in that the chamber is evacuated to a pressure of from about 66.5 to 130 μbar (0.05 to 0.1 torr).

3. Process according to claim 1 or 2, characterized in that the concentration of hydrogen peroxide in the solution is from about 10 to 70 % by weight.

4. Process according to claim 3, characterized in that the concentration in the solution is from about 30 to 50 % by weight.

5. Process according to any one of claims 1 to 4, characterized in that the period of time in step c) is less than 1 hour.

6. Process according to any one of claims 1 to 5, characterized in that the chamber is maintained at room temperature during the sterilization process.

**Revendications**

1. Un procédé pour la stérilisation à la vapeur d'articles comportant les étapes de :
   a) disposer un article devant être stérilisé dans une chambre,
   b) mettre sous vide ladite chambre,
   c) créer une atmosphère de peroxyde d'hydrogène dans la chambre et laisser la vapeur de peroxyde d'hydrogène en contact avec l'article,
   d) maintenir la chambre sous basse pression pendant une période de temps suffisante pour réaliser la stérilisation,
   caractérisé en ce que :
   - la chambre est mise sous vide jusqu'à une pression inférieure à 1,33 mbar (1,0 torr),
   - le peroxyde d'hydrogène est introduit dans la chambre sous la forme d'une solution aqueuse,
   - la concentration de vapeur de peroxyde d'hydrogène en contact avec l'article se situe dans la gamme d'environ 0,1 à environ 10 mg/l, et
   - la chambre est maintenue à une température inférieure à environ 40°C et à une pression inférieure à la tension de vapeur du peroxyde d'hydrogène pendant le procédé de stérilisation.

2. Procédé suivant la revendication 1, caractérisé en ce que la chambre est mise sous vide jusqu'à une pression d'environ 66,5 à 130 $\mu$bars (0,01 à 0,1 torr).

3. Procédé suivant la revendication 1 ou 2, caractérisé en ce que la concentration en peroxyde d'hydrogène dans la solution se situe entre environ 10 et 70 % en poids.

4. Procédé suivant la revendication 3, caractérisé en ce que la concentration dans la solution est d'environ 30 à 50 % en poids.

5. Procédé suivant l'une quelconque des revendications 1 à 4, caractérisé en ce que la période de temps pendant l'étape c) est inférieure à 1 heure.

6. Procédé suivant l'une quelconques des revendications 1 à 5, caractérisé en ce que la chambre est maintenue à la température ambiante pendant le procédé de stérilisation.

**Patentansprüche**

1. Verfahren zur Dampfsterilisation von Gegenständen mit den folgenden Stufen:
   a) Einbringen eines zu sterilisierenden Gegenstands in eine Sterilisationskammer,
   b) Evakuieren der genannten Kammer,
   c) Herstellen einer Wasserstoffperoxid-Atmosphäre in der Kammer und in Berührung kommen lassen des Gegenstands mit dem Wasserstoffperoxid-Dampf,
   d) Halten der Kammer bei einem niedrigen Druck während einer genügend langen Zeit, um die Sterilisation zu erreichen,
   dadurch gekennzeichnet, daß
   - die Kammer auf einen Druck unter 1,33 mbar (1,0 Torr) evakuiert wird,
   - das Wasserstoffperoxid in die Kammer in Form einer wäßrigen Lösung eingeführt wird,
   - die Konzentration des den Gegenstand kontaktierenden Wasserstoffperoxid-Dampfs im Bereich von etwa 0,1 bis etwa 10 mg/l ist, und
   - die Kammer bei einer Temperatur unter etwa 40°C und einem Druck unter dem Dampfdruck des Wasserstoffperoxids während des Sterilisationsverfahrens gehalten wird.

2. Verfahren nach Anspruch 1, dadurch gekennzeichnet, daß die Kammer auf einen Druck von etwa 66,5 bis 130 $\mu$bar (0,05 bis 0,1 Torr) evakuiert wird.

3. Verfahren nach Anspruch 1 oder 2, dadurch gekennzeichnet, daß die Konzentration des Wasserstoffperoxids in der Lösung von etwa 10 bis 70 Gew.-% beträgt.

4. Verfahren nach Anspruch 3, dadurch gekennzeichnet, daß die Konzentration in der Lösung etwa 30 bis 50 Gew.-% beträgt.

5. Verfahren nach einem der Ansprüche 1 bis 4, dadurch gekennzeichnet, daß die Zeitdauer in Stufe c) weniger als eine Stunde beträgt.

6. Verfahren nach einem der Ansprüche 1 bis 5, dadurch gekennzeichnet, daß die Kammer bei Raumtemperatur während des Sterilisationsverfahren gehalten wird.

9

## FIG-1

## FIG-2

# FIG-3